# DEMANDE DE BREVET EUROPEEN

(11) **EP 1 637 187 A1**
(43) Date de publication de la demande: **22.03.2006**
(21) Numéro de dépôt: 05300729.0
(22) Date de dépôt: 08.09.2005
(51) Int. Cl.: A61Q 1/04, A61Q 1/10, A61Q 3/02, A61Q 5/02, A61Q 5/04, A61Q 5/06, A61Q 5/08, A61Q 5/12, A61Q 9/02, A61K 8/30, A61K 8/31, A61K 8/33, A61K 8/40, A61K 8/49

(54) **Composition cosmétique comprenant au moins un diamantoïde, destinée à renforcer les propriétés mécaniques de certains matériaux**

(30) Priorité: 09.09.2004 FR 0452008
(71) Demandeur: L'OREAL, 75008 Paris (FR)
(72) Inventeur: Mathonneau, Estelle, 75010, PARIS (FR)
(74) Mandataire: Catherine, Alain

(57) **Abrégé**

L'invention concerne une composition cosmétique comprenant, avec un milieu cosmétiquement acceptable, au moins un diamantoïde constitué d'au moins deux unités adamantanes, ainsi que l'utilisation cosmétique de ces diamantoïdes pour renforcer les propriétés mécaniques de certains matériaux utilisés en cosmétique.

## Description

La présente invention est relative à de nouvelles compositions cosmétiques, comprenant au moins un diamantoïde, ainsi qu'à des procédés de traitement des matières kératiniques consistant à appliquer une composition cosmétique comprenant au moins un diamantoïde.

La plupart des produits de coiffage permettant de fixer la chevelure contiennent des polymères soit rigides soit élastiques. Il existe un besoin permanent d'amélioration des propriétés mécaniques de ces polymères. De nombreux types de matériaux ont été utilisés, les plus courant sont les charges.

En maquillage et soins, il existe aussi beaucoup d'associations avec des charges permettant d'améliorer les propriétés mécaniques de polymères utilisés dans les compositions. Ces charges sont le plus souvent inorganiques : la silice est la plus couramment utilisée.

De plus, la plus part des produits de coiffage permettant d'améliorer le volume de la chevelure sont à base de polymères filmogènes. L'inconvénient lié à ce type d'approche réside dans le fait que l'effet cosmétique disparaît dès le premier shampoing et que le toucher du cheveux est altéré (toucher chargé). On connaît par ailleurs les traitements permanents des fibres kératiniques, donnant des touchers moins chargés. Ces traitements utilisent un agent réducteur et un agent oxydant et nécessitent une mise sous tension mécanique des cheveux à l'aide d'un matériel d'enroulage, afin de conférer une mise en forme durable de la chevelure.

Bien qu'ils permettent effectivement d'augmenter le volume de la chevelure, ces procédés présentent l'inconvénient de modifier la forme de la coiffure et le niveau de frisure, et de dégrader la nature le toucher de la fibre. L'utilisation de nanotubes de carbone pour augmenter le volume de la chevelure, sans pour autant charger le toucher sur les cheveux, a été proposé notamment dans la demande de brevet français FR 2 840 529. Ils présentent l'avantage de ne pas augmenter la viscosité du milieu. Les nanotubes peuvent, cependant, parfois être difficiles à véhiculer sur les substrats et le support cosmétique doit être choisi avec soin, ce qui peut parfois limiter le type d'applications possibles. Ils sont par ailleurs difficilement fonctionnalisables.

Il apparaît ainsi nécessaire de développer des compositions permettant de renforcer les propriétés mécaniques de certains matériaux utilisés en cosmétique sans pour autant modifier l'aspect de la composition, et en particulier sans charger le toucher sur les cheveux.

La présente invention propose de nouvelles compositions cosmétiques permettant de remédier à ces inconvénients.

En particulier, la Demanderesse vient de découvrir de façon surprenante qu'une composition comprenant des diamantoïdes permet de renforcer les propriétés mécaniques de certains matériaux utilisés en cosmétique, et en particulier dans le maquillage, le soin de la peau, le soin des cheveux, le coiffage-coloration, ou encore l'hygiène corporelle. Les principaux matériaux visés sont les polymères.

La présente invention a donc pour objet une composition cosmétique comprenant au moins un diamantoïde.

L'invention a également pour un procédé de traitement des matières kératiniques consistant à appliquer une composition cosmétique telle que définei ci-dessus.

D'autres objets de l'invention apparaîtront à la lecture de la description et des exemples qui suivent.

L'invention a pour objet une composition cosmétique comprenant dans un milieu cosmétiquement acceptable au moins un diamantoïde constitué d'au moins deux unités adamantanes.

On entend par diamantoïde un hydrocarbure de taille nanométrique à l'état solide. Le diamantoïde le plus simple est constitué de 10 atomes de carbone, il s'agit de l'adamantane (C₁₀H₁₆). L'adamantane constitue une « unité » pour obtenir les diamantoïdes également désignés sous le terme polyadamantane (5 unités adamantanes). A partir du pentamantane, les formes du diamantoïde peuvent varier. On trouve ainsi des formes hélicoïdales, ou encore en forme de cube ou de cylindre. Les diamantoïdes ont une forte rigidité puisque certains atomes de carbone ont un conformation tétragonale équivalente à la conformation du carbone dans le diamant, mais également une bonne stabilité thermique et de bonnes propriétés de transparence de par leur taille.

Les diamantoïdes peuvent être classiquement obtenus par séparation à partir d'hydrocarbures tels que le gaz naturel. Des procédés d'extraction de diamantoides à partir du gaz naturel ont été décrits dans les brevets US5,019,665 ; US5,461,184 et US5,498,812. Ces procédés consistent à solubiliser les diamantoïdes contenus dans le gaz naturel grâce à des solvants appropriés, puis à séparer les diamantoides du solvant.

Les unités adamantanes constituant les diamantoïdes, peuvent comprendre un ou plusieurs hétéroatomes choisis parmi les éléments des colonnes III, IV, V et VI du tableau périodique autres que le carbone.

Des diamantoïdes comprenant des hétéroatomes, ainsi que leurs procédés de synthèse ont été décrits dans les brevets ou demandes de brevet US5,367,097 , et US2004/0059145 et dans les demandes internationales WO9428885 et W02004/01512.

Selon un mode de réalisation de l'invention, afin d'obtenir une solubilisation ou des propriétés optimales des diamantoïdes dans le milieu cosmétique considéré, ces derniers peuvent être fonctionnalisés.

Par l'expression « fonctionnalisé », on entend selon l'invention la présence de groupements fonctionnels pouvant interagir physiquement ou chimiquement entre eux ou avec le milieu extérieur.

Tous les mécanismes réactionnels peuvent être utilisés pour fonctionnaliser les unités adamantanes constituant les diamantoïdes de l'invention. A titre d'exemple la fonctionnalisation des diamantoïdes peut être réalisée par un mécanisme réactionnel du type substitution nucléophile, substitution électrophile, substitution radicalaire, addition, élimination, réarrangement, oxydation, réduction, réaction acido-basique, réaction électrochimique ou encore réaction photochimique.

Chaque unité adamantane peut comprendre 1 à 10 groupements fonctionnels identiques ou différents.

Parmi les fonctions pouvant être utilisées pour obtenir les diamantoïdes fonctionnalisés, on peut citer les groupements acides carboxyliques, les amides amines, esters, nitrites, hydroxy, thiols, alkyle, alkényle, aryle. Ces groupements peuvent être également choisis parmi les oligomères, les polymères, et les dendrimères.

Le cas échéant, ces réactions peuvent porter des substitutions sur les atomes mutivalents.

Outre l'amélioration de la dispersion des diamantoïdes dans les milieux cosmétiques, la fonctionnalisation des unités adamantanes peut également être réalisée afin d'augmenter l'affinité des diamantoïdes pour la matière kératinique.

L'amélioration de l'affinité entre les diamantoïdes et la matière kératinique induite par la fonctionnalisation peut être le fruit de l'accroissement des interactions de type Van der Waals, et/ou le fruit de l'apparition de liaisons hydrogènes et/ou ioniques. Ainsi le ou les groupements fonctionnels sont susceptibles de créer avec certains matériaux, et en particulier avec les fibres kératiniques, une ou plusieurs liaisons choisies parmi les interactions de type Van der Waals, les liaisons hydrogènes, les liaisons ioniques et les liaisons covalentes.

Dans ce cadre, on peut citer l'arylation des diamantoïdes décrite dans la demandes de brevet US5347063, le couplage de diamantoïdes avec des cycles aromatiques décrite dans la demande de brevet US5,369,213, mais également, une fonctionalisation par des amines, comme cela est décrit dans la demande de brevet US5,380,947.

Des diamantoïdes fonctionalisés convenant à la réalisation de la présente invention ont également été décrits dans les demandes internationales WO02/057201 et WO03/050066.

Les diamantoïdes substitués peuvent également être polymérisés. Des réactions de polymérisation de diamantoides substitués sont décrits en particulier dans les demandes US5,053,434, WO92/13909 et WO02/057201.

Pour accroître l'affinité des diamantoïdes pour la matière kératinique, il est également possible de greffer de façon covalente les unités adamantanes sur la fibre kératinique via des groupements réactifs.

Pour ce faire, la fonctionnalisation est en effet réalisée à l'aide de groupements possédant une certaine réactivité sur les acides aminés constituant la matière kératinique.

De préférence le ou les groupements fonctionnels susceptibles de créer avec les fibres kératiniques une ou plusieurs liaisons chimiques covalentes, sont choisis parmi les groupements susceptibles de réagir avec les thiols, les disulfures, les acides carboxyliques, les alcools et les amines.

De préférence, le ou les groupements fonctionnels susceptibles de réagir avec les thiols, les disulfures, les acides carboxyliques, les alcools et les amines sont choisis parmi :
- les époxydes,
- les groupements à cycle aziridine,
- les groupements vinyle et vinyle activé, tels que l'acrylonitrile, les esters acryliques et méthacryliques, l'acide et l'ester crotonique, les acides et esters cinnamiques, le styrène et ses dérivés, le butadiène, les éthers de vinyle, les vinyles cétones, les esters maléiques, les maléimides, les vinyles sulfones, le vinyle cyanoacrylate,
- les acides carboxyliques et leurs dérivés comme les fonctions anhydride, chlorure d'acide, esters,
- les acétals, hémiacétals,
- les aminals, hémiaminals,
- les cétones, alpha-hydroxycétones, alpha-halocétones,
- les lactones, thiolactones,
- l'isocyanate,
- le thiocyanate,
- les imines,
- les imides (succinimides, glutimides), tels que le N-hydroxysuccimide ester,
- les imidates,
- l'oxazine et l'oxazoline,
- l'oxazinium et l'oxazolinium,
- les halogénures d'alkyle ou d'aryle ou d'aralkyle, l'halogène étant choisi parmi l'iode, le brome ou le chlore,
- les halogénures de cycle insaturé, le cycle étant un cycle carboné ou un hétérocycle, tels que le chlorotriazine, le chloropyrimidine, le chloroquinoxaline, le chlorobenzotriazole,
- les halogénures de sulfonyle de formule RSO₂X, R étant un groupement alkyle, et X étant choisi parmi le fluor et le chlore,
- et les dérivés du silicium tels que les alcoxysilanes, et les sillanols.

Afin d'obtenir une dispersion (exfoliation) optimale des diamantoïdes dans le milieu cosmétique considéré, la composition contient de préférence au moins un tensio-actif.

Le tensio-actif peut être choisi parmi les molécules amphiphiles, les oligomères amphiphiles, les polymères amphiphiles et leurs mélanges. Ce tensio-actif peut être non ionique, anionique, cationique ou amphotère.

Les diamantoïdes peuvent avoir une taille de 1 nm à 1 mm.

De préférence, les diamantoïdes ont une masse moléculaire comprise entre 136 et 400g par mole.

Parmi les divers groupements hydrophobes des tensio-actifs, sont préférés les groupements capables de s'absorber par «π stating » (recouvrement des nuages électroniques des noyaux aromatiques) à la surface des unités adamantanes.

Parmi les molécules aptes à générer une telle interaction, sont préférées es molécules aromatiques telles que le styrène et ses dérivés ou le pyrène et ses dérivés.

Les groupements hydrophiles sont de préférence choisis afin d'améliorer l'affinité de la surface des nanotubes pour la matière kératinique que ce soit par l'accroissement des interactions de type Van der Waals et/ou la création de liaisons hydrogènes et/ou ioniques.

Les diamantoïdes peuvent être présent dans la composition cosmétique dans des proportions comprises entre 0,00001 et 30%, et de préférence entre 0,0001 et 10%, et encore de préférence entre 0,001% et 5% par rapport au poids total de la composition.

Le milieu cosmétiquement acceptable comprenant les diamantoïdes est de préférence constitué par :
- de l'eau,
- des alcools aliphatiques ou aromatiques, de préférence l'éthanol, l'alcool benzylique, les alcools gras, les polyols modifiés ou non, tels que le glycérol, le glycol, le propylène glycol, le dipropylène glycol, le butylène glycol, le butyle diglycol,
- des silicones, volatiles ou non,
- des huiles minérales, organiques ou végétales,
- des cires oxyéthylénées ou non, des paraffines, des alcanes et de préférence des alcanes de C₅ à C₁₀,
- des acides gras, des amines grasses, des esters gras et plus particulièrement des benzoates ou des salicylates d'alcool gras,
- de l'acétone, de la méthyléthylcétone, de l'acétate de méthyle, de l'acétate de butyle, de l'acétate d'éthyle, du diméthoxyéthane, du diéthoxyéthane,
- et leurs mélanges.

Le milieu cosmétiquement acceptable peut se présenter tel quel ou sous forme d'émulsionnée ou de dispsersion. Les diamantoïdes ou certains agents de la composition peuvent être encapsulés. Les compositions cosmétiques selon l'invention peuvent en outre contenir un propulseur, comme dans le cas des sprays.

De préférence, le propulseur est constitué par des gaz comprimés ou liquéfiés choisis parmi l'air, le gaz carbonique, l'azote comprimé, un gaz soluble comme le diméthyléther, les hydrocarbures halogénés, en particulier fluorés ou non, et leurs mélanges.

La composition peut également contenir des additifs cosmétiques usuels choisis parmi les silicones sous forme soluble, dispersés, notamment micro ou nano-dispersés, les agents épaississants, les agents conditionneurs, les agents adoucissants, les agents anti-mousse, les agents hydratants, les agents émollients, les plastifiants, les filtres solaire hydrosolubles et liposolubles, siliconés ou non siliconés, les colorants permanents ou temporaires, les pigments minéraux ou organiques, colorés ou non colorés, les charges minérales, les argiles, les nacres, les opacifiants, les colloïdaux, les parfums, les peptisants, les conservateurs, les céramides, les pseudo-céramides, les vitamines et les pro-vitamines dont le penténol, les protéines, les agents séquestrants, les agents solubilisants, les agents alcalinisants, les agents anti-corrosion, les corps gras tels que les huiles végétales, animales, minérales et synthétiques, les agents réducteurs ou anti-oxydants, les agents oxydants, et leurs mélanges.

La composition cosmétique décrite ci-dessus peut être rincée ou non rincée, et peut se présenter sous diverses formes galéniques.

En particulier, la composition cosmétique peut être une composition pour application capillaire, se présentant sous la forme d'une lotion, d'un spray aérosol ou non, d'une mousse, d'un gel, d'une pâte, d'une crème, d'un stick, d'un shampoing, d'un après-shampoing ou d'un soin, d'une composition décoloration ou d'une composition pour permanente, d'une composition de coloration ou encore d'une coloration de défrisage.

Alternativement, la composition cosmétique peut être une composition pour application sur la peau se présentant sous la forme d'un gel, d'un spray aérosol ou non, d'une mousse, d'une crème, d'un rouge à lèvres, d'un eye-liner, d'un mascara, d'un blush, d'un vernis à ongles, d'une mousse de rasage, ou d'une poudre.

L'invention a également pour objet un procédé de traitement des matières kératiniques consistant à appliquer une composition cosmétique telle que définie ci-dessus.

Enfin, l'invention a aussi pour objet l'utilisation cosmétique d'au moins un diamantoïde pour renforcer les propriétés mécaniques d'au moins un polymère.

L'invention pourra être mieux comprise à l'aide de l'exemple non limitatif qui suit et qui constitue un mode de réalisation préférentiel des compositions selon l'invention.

### Exemple 1 : Composition de coiffage selon l'invention

| | |
|---|---|
| Cyclopentasiloxane¹ | 10% |
| Huile de ricin hydrogénée oxyéthylénée (7OE)² | 10% |
| Diméthicone copolyol³ | 0,5% |
| Propylène glycol | 2,5% |
| Behentrimonium chloride⁴ | 1,2% |
| Diamantoïdes substitués par des hydroxyles⁵ | 0,01 % |
| Eau | qsp 100% |

| | |
|---|---|
| (1) DC 245 FLUID commercialisé par DOW CORNING | |
| (2) ARLACEL 989 commercialisé par UNIQUEMA | |
| (3) DC 2-5225C commercialisé par DOW CORNING | |
| (4) GENAMIN KDM-F commercialisé par HOECHST | |
| (5) Diamantoïdes tels que décrits dans le demande WO 02/057201 | |

## Revendications

1. Composition cosmétique **caractérisée en ce qu'**elle comprend, en association avec un milieu cosmétiquement acceptable, au moins un diamantoïde constitué d'au moins deux unités adamantanes.

2. Composition cosmétique selon la revendication 1, **caractérisée en ce que** les unités adamantanes comprennent un ou plusieurs hétéroatomes choisis parmi les éléments du tableau périodique III, IV, V et VI et à la place d'un ou plusieurs atomes de carbone.

3. Composition cosmétique selon la revendication 1 ou 2, **caractérisée en ce que** le diamantoïde est fonctionnalisé.

4. Composition cosmétique selon l'une quelconque des revendications 1 à 3, **caractérisé en** en ce que chaque unité adamantane comprend 1 à 10 groupements fonctionnels identiques ou différents.

5. Composition cosmétique selon la revendication 4, **caractérisée en ce que** la fonctionnalisation des diamantoïdes est réalisée par un mécanisme réactionnel du type substitution nucléophile, substitution électrophile, substitution radicalaire, addition, élimination, réarrangement, oxydation, réduction, réaction acido-basique, réaction électrochimique ou réaction photochimique.

6. Composition cosmétique selon la revendication 4 ou 5, **caractérisée en ce que** la surface des diamantoïdes est fonctionnalisée par un ou plusieurs groupements choisis parmi les groupements acides carboxyliques, les amides amines, esters, nitrites, hydroxy, thiols, alkyle, alkényle, aryle. Ces groupements peuvent être également choisis parmi les oligomères, les polymères, et les dendrimères.

7. Composition cosmétique selon la revendication 4, **caractérisée en ce que** le ou les groupements fonctionnels sont susceptibles de créer avec les fibres kératiniques une ou plusieurs liaisons choisies parmi les interactions de type Van der Waals, les liaisons hydrogènes, les liaisons ioniques et les liaisons covalentes.

8. Composition cosmétique selon la revendication 4, **caractérisée en ce que** le ou les groupements fonctionnels susceptibles de créer avec les fibres kératiniques une ou plusieurs liaisons chimiques covalentes sont choisis parmi les groupements susceptibles de réagir avec les thiols, les disulfures, les acides carboxyliques, les alcools et les amines.

9. Composition cosmétique selon la revendication 8, **caractérisée en ce que** le ou les groupements fonctionnels susceptibles de réagir avec les thiols, les disulfures, les acides carboxyliques, les alcools et les amines sont choisis parmi :
- les époxydes,
- les groupements à cycle aziridine,
- les groupements vinyle et vinyle activé, tels que l'acrylonitrile, les esters acryliques et méthacryliques, l'acide et l'ester crotonique, les acides et esters cinnamiques, le styrène et ses dérivés, le butadiène, les éthers de vinyle, les vinyles cétones, les esters maléiques, les maléimides, les vinyles sulfones, le vinyle cyanoacrylate,
- les acides carboxyliques et leurs dérivés comme les fonctions anhydride, chlorure d'acide, esters,
- les acétals, hémiacétals,
- les aminals, hémiaminals,
- les cétones, alpha-hydroxycétones, alpha-halocétones,
- les lactones, thiolactones,
- l'isocyanate,
- le thiocyanate,
- les imines,
- les imides (succinimides, glutimides), tels que le N-hydroxysuccimide ester,
- les imidates,
- l'oxazine et l'oxazoline,
- l'oxazinium et l'oxazolinium,
- les halogénures d'alkyle ou d'aryle ou d'aralkyle, l'halogène étant choisi parmi l'iode, le brome ou le chlore,
- les halogénures de cycle insaturé, le cycle étant un cycle carboné ou un hétérocycle, tels que le chlorotriazine, le chloropyrimidine, le chloroquinoxaline, le chlorobenzotriazole,
- les halogénures de sulfonyle de formule RSO₂X, R étant un groupement alkyle, et X étant choisi parmi le fluor et le chlore,
- et les dérivés du silicium tels que les alcoxysilanes, et les sillanols.

10. Composition cosmétique selon l'une quelconque des revendication 1 à 9, **caractérisée en ce que** les diamantoïdes ont une taille de 1 nm à 1 mm.

11. Composition cosmétique selon l'une quelconque des revendications 1 à 10, **caractérisée en ce que** les diamantoïdes ont une masse moléculaire comprise entre 136 et 400gr par mole.

12. Composition cosmétique selon l'une quelconque des revendications 1 à 11, **caractérisée en ce que** les diamantoïdes sont présents dans la composition cosmétique dans des proportions en poids comprises entre 0,00001 et 30%, et de préférence entre 0,0001 et 10%, et de manière tout à fait préférée entre 0,001 % et 5% par rapport au poids total de la composition.

13. Composition cosmétique selon l'une quelconque des revendications 1 à 12, **caractérisée en ce qu'**elle comprend au moins un tensio-actif.

14. Composition cosmétique selon la revendication 13, **caractérisée en ce que** le ou les tensio-actifs sont choisis parmi les molécules amphiphiles, les oligomères amphiphiles, les dendrimères amphiphiles, les polymères amphiphiles et leurs mélanges.

15. Composition cosmétique selon l'une quelconque des revendications 1 à 14, **caractérisée en ce que** le milieu cosmétiquement acceptable comprenant les diamantoïdes est constitué par :
- de l'eau,
- des alcools aliphatiques ou aromatiques, de préférence l'éthanol, l'alcool benzylique, les alcools gras, les polyols modifiés ou non, tels que le glycérol, le glycol, le propylène glycol, le dipropylène glycol, le butylène glycol, le butyle diglycol,
- des silicones, volatiles ou non,
- des huiles minérales, organiques ou végétales,
- des cires oxyéthylénées ou non, des paraffines, des alcanes et de préférence des alcanes de C₅ à C₁₀,
- des acides gras, des amines grasses, des esters gras et plus particulièrement des benzoates ou des salicylates d'alcool gras,
- de l'acétone, de la méthyléthylcétone, de l'acétate de méthyle, de l'acétate de butyle, de l'acétate d'éthyle, du diméthoxyéthane, du diéthoxyéthane, et leurs mélanges.

16. Composition cosmétique selon l'une quelconque des revendications 1 à 15, **caractérisée en ce que** la composition contient en outre un propulseur.

17. Composition cosmétique selon la revendication 16, **caractérisée en ce que** le propulseur est constitué par un gaz comprimé ou liquéfié choisi parmi l'air, le gaz carbonique, l'azote comprimé, un gaz soluble comme le diméthyléther, les hydrocarbures halogénés, en particulier fluorés ou non, et leurs mélanges.

18. Composition cosmétique selon l'une quelconque des revendications 1 à 17, **caractérisée en ce que** la composition contient également des additifs cosmétiques choisis parmi les silicones sous forme soluble, dispersés, notamment micro ou nano-dispersés, les agents épaississants, les agents tensio-actifs non ioniques, anioniques, cationiques et amphotères, les agents conditionneurs, les agents adoucissants, les agents anti-mousse, les agents hydratants, les agents émollients, les plastifiants, les filtres solaire hydrosolubles et liposolubles, siliconés ou non siliconés, les colorants permanents ou temporaires, les pigments minéraux ou organiques, colorés ou non colorés, les charges minérales, les argiles, les nacres, les opacifiants, les colloïdaux, les parfums, les peptisants, les conservateurs, les céramides, les pseudo-céramides, les vitamines et les pro-vitamines dont le penténole, les protéines, les agents séquestrants, les agents solubilisants, les agents alcalinisants, les agents anti-corrosion, les corps gras tels que les huiles végétales, animales, minérales et synthétiques, les agents réducteurs ou anti-oxydants, les agents oxydants, et leurs mélanges.

19. Composition cosmétique selon l'une quelconque des revendications 1 à 18, **caractérisée en ce que** la composition est une composition pour application capillaire, se présentant sous la forme d'une lotion, d'un spray aérosol ou non, d'une mousse, d'un gel, d'une pâte, d'une crème, d'un stick, d'un shampoing, d'un après-shampoing, ou d'un soin, d'une composition de décoloration ou d'une composition pour permanente ou défrisage.

20. Composition cosmétique selon l'une quelconque des revendications 1 à 18, **caractérisée en ce que** la composition est une composition pour application sur la peau se présentant sous la forme d'un gel, d'un spray aérosol ou non, d'une mousse, d'une crème, d'un rouge à lèvres, d'un eye-liner, d'un mascara, d'un blush, d'un vernis à ongles, d'une mousse de rasage, ou d'une poudre.

21. Procédé de traitement des matières kératiniques consistant à appliquer une composition cosmétique selon l'une quelconque des revendications 1 à 20.

22. Utilisation en cosmétique d'au moins un diamantoïde .

23. Utilisation selon la revendication 22 pour renforcer les propriétés mécaniques d'au moins un polymère.
